# EUROPEAN PATENT APPLICATION

(11) **EP 4 070 769 A1**
(43) Date of publication of application: **12.10.2022**
(21) Application number: 21167123.5
(22) Date of filing: 07.04.2021
(51) Int. Cl.: A61F 13/15, A61F 13/49

(54) **AN ABSORBENT SANITARY ARTICLE AND A METHOD FOR PRODUCING THE SAME**

(71) Applicant: Fameccanica.Data S.p.A., 66020 San Giovanni Teatino (CH) (IT)
(72) Inventor: SABLONE, Gabriele, I-66020 San Giovanni Teatino (Chieti) (IT)
(74) Representative: Marchitelli, Mauro

(57) **Abstract**

An absorbent sanitary article comprising an absorbent body (12) having a front panel (38) of a loop material attached to the backsheet (15) in a front portion (76) of the absorbent body (12), and two elastic waistbands (28) having respective rear oblique sides (24) fixed to corresponding rear oblique sides (22) of the absorbent body (12), and having respective micro-hook fastening elements (34), fixed at respective end portions (36) of the elastic waistbands (28) opposite to the rear oblique sides (24).

## Description

### Field of the invention

The present invention relates to an absorbent sanitary article.

The invention relates in particular to absorbent sanitary articles having an elastic waistband that can be opened and reclosed around the waist of the user.

Embodiments of the invention relate to a method for producing absorbent sanitary articles according to the so-called "Machine Direction" production technique.

### Prior art

Absorbent sanitary articles having elastic waistbands are normally formed by an absorbent body having an elongated shape along a longitudinal axis, and two transverse waistbands (a front waistband and a rear waistband) fixed to opposite ends of the absorbent body. The transverse waistbands may be elastic and can be fixed to each other permanently or by refastenable closure formations.

For producing absorbent sanitary articles composed of an absorbent body with an elongated shape and two transverse waistbands, a production technique called "Cross Direction" is normally used, which consists of forming two continuous elastic parallel bands forming the front and rear waistbands, which advance in a machine direction and applying the absorbent bodies transversally between the continuous elastic bands. The absorbent bodies oriented transversely with respect to the machine direction are fixed at their opposite ends to the continuous bands forming the waistbands.

The absorbent sanitary articles manufactured in "Cross Direction" typically have closure formations (welds or hook-and-loop refastenable elements) on the elastic bands which in use are positioned on the hips of the user, which may be a problem, especially in case of absorbent products for adults, since the closure formations on the hips may be visible under the garments.

WO2000/047151 A1 describes a method for producing absorbent sanitary articles according to the Machine Direction production technique. The method described in this document envisages the formation of a continuous absorbent composite tape that advances in a machine direction, in the direction of its longitudinal axis. Two continuous elastic bands are overlapped on respective longitudinal side edges of the continuous absorbent composite tape. The continuous elastic bands are fixed to the continuous absorbent composite tape along oblique junction lines with a V-shape open outwards. The method involves cutting the continuous composite tape and the elastic bands along cutting lines in the shape of a double Y, so as to give rise to discrete absorbent sanitary articles each consisting of an absorbent body and two longitudinal elastic bands fixed at opposite ends of the absorbent body along oblique sides.

The solution known from WO2000/047151 provides absorbent sanitary articles which are permanently closed and shaped like pants. This solution cannot be used when refastenable absorbent sanitary articles are required, which are increasingly requested on the market by virtue of their greater convenience of use.

### Object and summary of the invention

The object of the present invention is to provide an absorbent sanitary article with elastic waistbands and a method for manufacturing the same, which overcome the problems of the prior art.

In particular, the present invention aims to provide a refastenable absorbent sanitary article with elastic waistbands which does not have closure formations on the hips of the user, and which has a simple structure and robust connections between the elastic waistbands and the absorbent body.

According to the present invention, this object is achieved by an absorbent sanitary article having the features of claim 1.

According to another aspect, the present invention relates to a method for manufacturing absorbent sanitary articles in Machine Direction having the features of claim 7.

The claims form an integral part of the disclosure provided here in relation to the invention.

### Brief description of the drawings

The present invention will now be described in detail with reference to the attached drawings, given purely by way of non-limiting example, wherein:
- Figure 1 is a plan view of an absorbent sanitary article according to the present invention in an extended configuration,
- Figure 2 is a perspective view of the absorbent sanitary article of Figure 1 in a configuration of use,
- Figure 3 is a plan view of the absorbent sanitary article of Figures 1 and 2 in an extended position and with the elastic bands open,
Figures 4-10 are schematic plan views showing various steps of a method for producing absorbent sanitary articles.

It will be appreciated that the various figures may not be represented on the same scale. It will also be appreciated that in some figures certain elements or components may not be shown for a better understanding.

### Detailed description

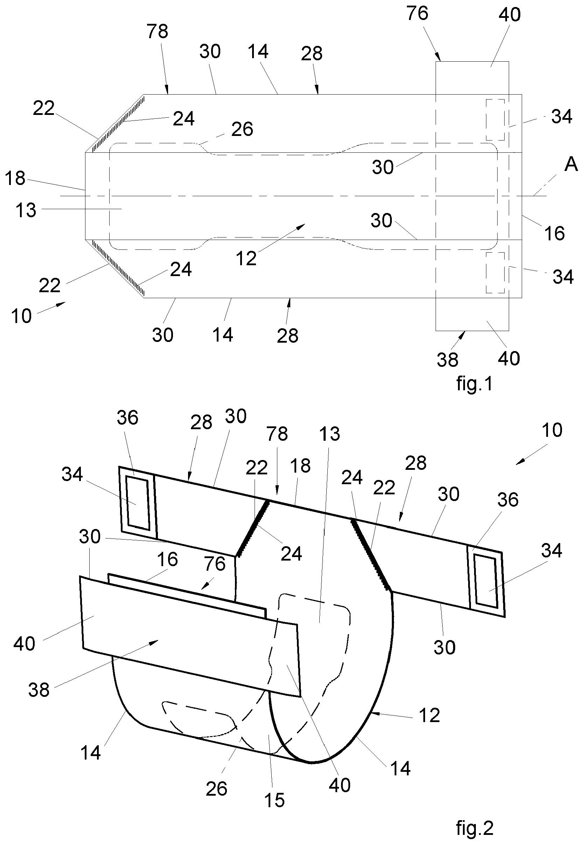

With reference to Figures 1-3, numeral 10 indicates an absorbent sanitary article according to an embodiment of the present invention. The absorbent sanitary article 10 may be a training pant, an incontinence absorbent article for adults, or the like.

The absorbent sanitary article 10 comprises an absorbent body 12 elongated along a longitudinal axis A. The absorbent body 12 has two longitudinal sides 14 that extend along a direction generally parallel to the longitudinal axis A. The longitudinal sides 14 may be straight, as illustrated by way of example in the figures, or they may be shaped in different ways. For instance, the longitudinal sides 14 may be curved, with a curvature that in use follows the curves of the legs.

The absorbent body 12 comprises a front portion 76 having a front transverse side 16 and a rear portion 78 having a rear transverse side 18. The front and rear transverse sides 16, 18 may be orthogonal to the longitudinal axis A.

The absorbent body 12 has two rear oblique sides 22 which are inclined to the longitudinal axis A. The two rear oblique sides 22 may be symmetrical with respect to the longitudinal axis A. Each of the oblique rear sides 22 connects one end of the respective rear transverse side 18 to a corresponding end of the respective longitudinal side 14.

The absorbent body 12 may comprise an absorbent core 26, which may be sandwiched between a topsheet 13 of liquid-permeable material, typically made of a non-woven web, and a backsheet 15 of impermeable material typically made of a film of plastic material, e.g. of polyethylene. The topsheet 13 of the absorbent body 12 in use is in contact with the user's body, whereas the backsheet 15 in use faces away from the user's body.

Additional absorbent layers may be comprised between the topsheet 13 and the backsheet 15, such as - for example - an Acquisition Diffusion Layer, commonly referred to as ADL. The absorbent body 12 may comprise additional components (not shown) as is customary in the field, such as, for example, leg cuffs applied onto the topsheet and extending parallel to the longitudinal axis A.

The absorbent sanitary article 10 comprises two elastic waistbands 28 elastically extendable in directions parallel to the longitudinal axis A. The two elastic waistbands 28 are located on opposite sides of the longitudinal axis A. Each of the two elastic waistbands 28 has two longitudinal sides 30 which, in the extended position of Figure 1, are parallel to the longitudinal axis A. Each of the two elastic waistbands 28 has a rear oblique side 24 overlapped and fixed to a corresponding rear oblique side 22 of the absorbent body 12. The rear oblique sides 24 of the two elastic waistbands 28 may be fixed to the rear oblique sides 22 of the absorbent body 12 by welding, e.g. by ultrasonic welding.

In a non-in-use extended configuration shown in Figure 1, the elastic waistbands 28 are in contact with the topsheet 13 of the absorbent body 12 and extend parallel to the longitudinal axis A between respective rear oblique sides 22 and the front transverse side 16 of the absorbent body 12.

The elastic waistbands 28 have respective fastening elements 34 fixed at respective end portions 36 opposite with respect to the rear oblique sides 24. The fastening elements 34 are fixed to respective inner surfaces of the elastic waistbands 28 which in the extended configuration of Figure 1 face the topsheet 13. In the extended configuration of Figure 1, the fastening elements 34 are releasably connected to corresponding portions of the topsheet 13. The fastening elements 34 may be micro-hook fastening elements of hook-and-loop fasteners of the type commonly referred to as Velcro^{™}.

In a possible embodiment, the fastening elements 34 may be fixed on non-elastic end portions 36 of the respective elastic waistbands 28. The fastening elements 34 may be fixed to the respective end portions 36 of the elastic waistbands 28 by glue or by welding. In a possible embodiment, the fastening elements 34 may be integrally formed on specific areas of non-woven layers of the elastic waistbands 28.

The absorbent sanitary article 10 may comprise a front panel 38 attached to the backsheet 15 in the front portion 76 of the absorbent body. The front panel 38 is made of a loop material capable of establishing a releasable and refastenable connection with the fastening elements 34. In a possible embodiment the front panel 38 may be made of a non-woven material. The front panel 38 may have fibers forming micro-loops capable of forming a hook-and-loop connection with the micro-hook fastening elements 34. The front panel 38 may be attached to the backsheet 15 by glue or by welding. In possible embodiments, the absorbent sanitary article 10 may not have a front panel of loop material and may have at least a part of the outer surface of the backsheet made of a material capable of establishing a releasable and refastenable connection with the fastening elements 34.

The front panel 38 may have a rectangular shape elongated in a direction transverse to the longitudinal axis A, with one of the major sides parallel and close to the front transverse side 16 of the absorbent body 12. The front panel 38 may have side portions 40 extending laterally beyond the longitudinal sides 14 of the absorbent body 12.

In a non-in use extended position the absorbent sanitary article 10 may be in the configuration of Figure 1. In a packaging condition the absorbent sanitary article may be folded around a central transverse line. The elastic waistbands 28 are in contact with the topsheet 13 and the fastening elements 34 are releasably attached to corresponding portions of the topsheet 13 adjacent to the front transverse side 16 of the absorbent body 12.

In use, the fastening elements 34 are detached from the topsheet 13, the elastic waistbands 28 are extended outwardly as shown in Figure 2 and the absorbent body 12 is bent in a U shape around the crotch of the user. Then, the elastic waistbands 28 are applied around the sides of the user and the fastening elements 34 are fastened to the front panel 38 so that the elastic waistbands 28 are closed around the waist of the user. The fastening elements 34 may be connected where desired on the front panel 38 so as to obtain a desired elastic tension of the elastic waistbands 28.

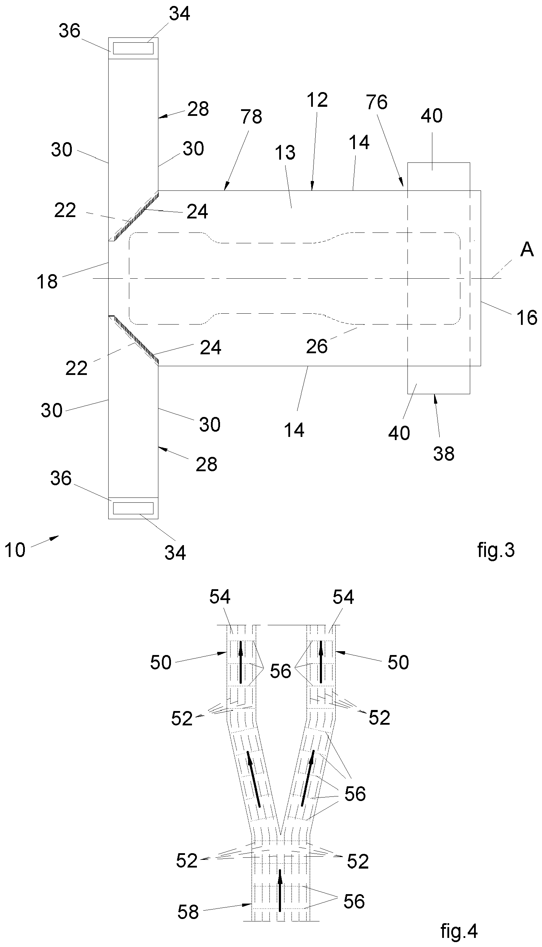

An embodiment of a method for producing an absorbent sanitary article 10 of the type illustrated in Figures 1-3 will now be described with reference to Figures 4-10.

With reference to figure 4, the method involves forming two continuous elastic bands 50 advancing parallel to each other in the respective longitudinal directions.

Each continuous elastic band 50 may comprise a plurality of parallel elastic threads 52 enclosed between two non-woven webs 54. The elastic threads 52 may be anchored to the two non-woven webs 54 while tensioned in the longitudinal direction. The elastic threads 52 may be anchored to the two non-woven webs 54 by a pattern of anchoring welds 56, as disclosed in EP-A-3092997 of the same Applicant. The anchoring welds may be arranged in lines transversal to the longitudinal axis of the continuous elastic bands 50 and spaced from each other in the longitudinal direction.

As shown in Figure 4, the two continuous elastic bands 50 may be obtained from a single continuous elastic band 58 which is cut longitudinally to form the two continuous elastic bands 54, which are spaced apart from each other in a transversal direction after the longitudinal cut.

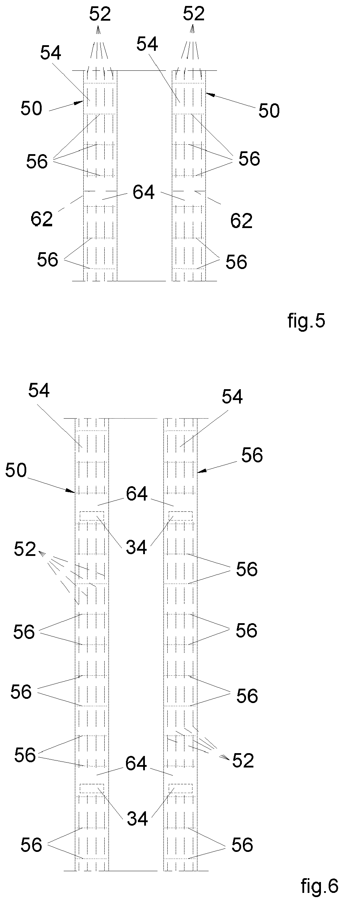

With reference to Figures 5, transverse cuts 62 may be formed on the two continuous elastic bands 50 at regular spacing along the longitudinal direction. The transverse cuts 62 may cut only the elastic threads 52, without cutting the non-woven webs 54. Following the transverse cuts 62, the elastic threads 52 retract in the longitudinal direction up to the transverse lines of anchoring welds 56 adjacent to the transverse cuts 62, thus forming on the elastic bands 50 non-elastic areas 64 spaced apart at regular intervals in the longitudinal direction.

With reference to Figures 6, discrete fastening elements 34 are fixed on respective non-elastic areas 64 of the two continuous elastic bands 50. Fixing of the fastening elements 34 on the continuous elastic bands 50 may be obtained by glue or by welding.

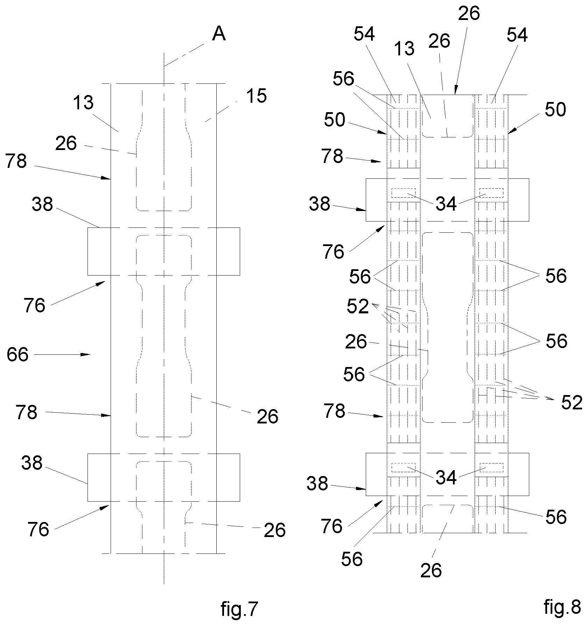

With reference to Figure 7, the method involves forming a continuous composite tape 66, comprising a plurality of discrete absorbent cores 26 aligned along a longitudinal axis A and sandwiched between a continuous topsheet 13 and a continuous backsheet 15. The continuous composite tape 66 has a front portion 76 and a rear portion 78 for each absorbent core 26.

A plurality of front panels 38 spaced apart from each other in the longitudinal direction are attached to the continuous backsheet 15 in respective front portions 76 of the continuous composite tape by glue or by welding.

With reference to Figure 8, the two continuous elastic bands 50 are overlapped to respective side portions of the continuous composite web 66 as it advances in the longitudinal direction A. The continuous elastic bands 50 are arranged in a predetermined phase relationship with respect to the continuous composite web 66. The fastening elements 34 of the two continuous elastic bands 50 face the continuous topsheet 13 of the continuous composite web 66 and are releasably connected thereto in respective front portions 76 of the continuous composite tape. Each pair of fastening elements 34 is opposite to a respective front panel 38.

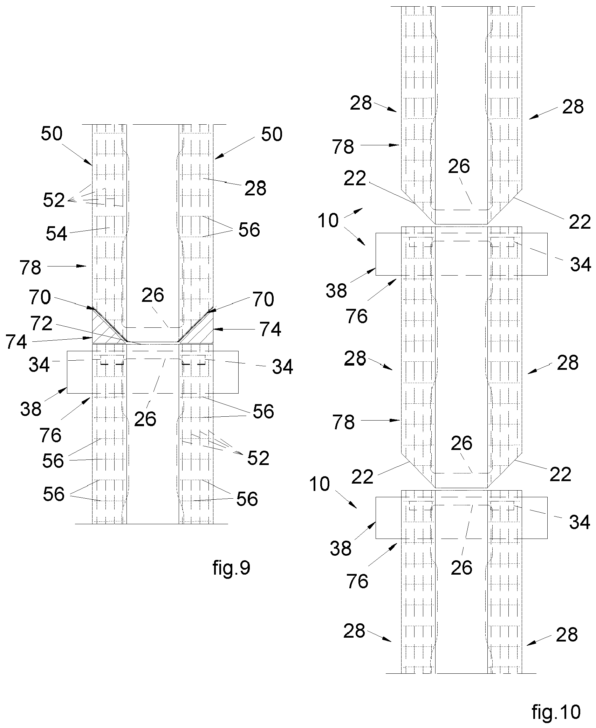

With reference to Figure 9, the two continuous elastic bands 50 are welded to the continuous composite tape 66 along inclined welding lines 70 spaced apart from each other in the longitudinal direction.

Then, the two continuous elastic bands 50 and the continuous composite tape 66 are cut along cutting lines 72. Each cutting line 72 has a transverse section which extends between two opposite longitudinal edges of the continuous composite tape 66, and two inclined sections parallel to the respective welding lines 70. Each cutting line 72 forms two triangular sections 74 adjacent to the respective welding lines 70 which are disposed as wastes.

With reference to Figure 10, after the cut along the cutting lines 72 the continuous composite tape 66 with the two continuous elastic bands 50 fixed thereto is split into a row of individual absorbent sanitary articles 10, each of which has the same structure shown in Figure 1.

Of course, without prejudice to the principle of the invention, the details of construction and the embodiments can be widely varied with respect to those described and illustrated, without thereby departing from the scope of the invention as defined by the claims that follow.

## Claims

1. An absorbent sanitary article comprising:
- an absorbent body (12) elongated along a longitudinal axis (A), having an absorbent core (26) sandwiched between a topsheet (13) and a backsheet (15), a front portion (76) having a front transverse side (16) and a rear portion (78) having a pair of rear oblique sides (22),
- two elastic waistbands (28) having respective rear oblique sides (24) overlapped and fixed to corresponding rear oblique sides (22) of the absorbent body (12), and having respective micro-hook fastening elements (34), suitable for establishing a hook-and-loop connection with said front panel (38), fixed at respective end portions (36) of the elastic waistbands (28) opposite to said rear oblique sides (24).

2. The absorbent sanitary article of claim 1, comprising a front panel (38) of a loop material attached to said backsheet (15) in said front portion (76) of the absorbent body (12).

3. The absorbent sanitary article of claim 1 or claim 2, wherein said front panel (38) has two side portions (40) which extends laterally outside the absorbent body (12).

4. The absorbent sanitary article of any of the preceding claims, wherein in a not-in-use extended position said two elastic waistbands (28) are in contact with said topsheet (13) and said fastening elements (34) are releasably connected to the topsheet (13) in the front portion (76) of said absorbent body (12).

5. The absorbent sanitary article of any of the preceding claims, wherein each of said elastic waistbands (28) comprises a plurality of elastic threads (52) sandwiched between two non-woven webs (54) and anchored to said non-woven webs (54).

6. The absorbent sanitary article of claim 5, wherein said absorbent body (12) has curved longitudinal sides (14).

7. A method for producing absorbent sanitary articles (10), comprising:
- providing a continuous composite tape (66) elongated along a longitudinal axis (A), having a plurality of absorbent cores (26) aligned to each other along said longitudinal axis (A) and sandwiched between a continuous topsheet (13) and a continuous backsheet (15), wherein the continuous composite tape (66) has - for each of said absorbent cores (26) - a front portion (76) and a rear portion (78),
- forming two continuous elastic bands (50) elongated along said longitudinal axis (A) and parallel to each other, each of said two continuous elastic bands (50) having a plurality of micro-hook fastening elements (34) spaced apart from each other along said longitudinal axis (A),
- overlapping said continuous elastic bands (50) on respective side portions of the continuous composite tape (66),
- fixing said continuous elastic bands (50) to the continuous composite tape (66) along inclined welding lines (70) inclined with respect to said longitudinal axis (A), wherein said inclined welding lines (70) are spaced apart from each other along said longitudinal axis (A) and are located in respective rear portions (78) of said continuous absorbent web (66),
- cutting the continuous absorbent web (66) and the continuous elastic bands (50) along cutting lines (72), so as to give rise to a succession of absorbent sanitary articles (10).

8. The method of claim 7, comprising applying said two continuous elastic bands (50) in contact with said continuous topsheet (13) and connecting said micro-hook fastening elements (34) to respective areas of the continuous topsheet (13) in respective front portions (76) of the continuous composite tape (66).

9. The method of claim 7 or claim 8, wherein each of said cutting lines (72) includes a transverse cutting line extending between two opposite longitudinal sides of said continuous composite tape (66) and two inclined cutting lines parallel to respective inclined welding lines (70).

10. The method of any of claims 7-9, comprising attaching a plurality of front panels (38) of a loop material to said backsheet (15) in said front portions (76) of the continuous composite tape (66).

11. The method of any of claims 7-10, wherein each of said continuous elastic bands (50) is formed by sandwiching a plurality of elastic threads (52) tensioned in a longitudinal direction between two non-woven webs (54) and anchoring said elastic threads (52) to said non-woven webs (54) in anchoring points spaced from each other in said longitudinal direction.

12. The method of claim 11, wherein said elastic threads (52) are anchored to said non-woven webs (54) by a pattern of anchoring welds (56).

13. The method of any of claims 7-12, comprising forming on said two continuous elastic bands (50) non-elastic end portions (36) spaced apart from each other along said longitudinal direction (A) and fixing said micro-hook fastening elements (34) to respective non-elastic end portions (36).

14. The method of claim 11 or claim 12, wherein said non-elastic end portions (36) are formed by making transverse cuts (62) on said continuous elastic bands (50) which cut said elastic threads (52) without cutting said non-woven webs (54).
